# EUROPÄISCHE PATENTSCHRIFT

(11) **EP 1 362 575 B1**
(45) Veröffentlichungstag und Bekanntmachung des Hinweises auf die Patenterteilung: **13.09.2006**
(21) Anmeldenummer: 03007858.8
(22) Anmeldetag: 05.04.2003
(51) Int. Cl.: A61Q 5/06, A61K 8/73, A61K 8/81

(54) **Wasserfreie Haarstylinggele**
Waterfree hair-styling gels
Gels de coiffage exempts d'eau

(30) Priorität: 02.05.2002 DE 10219597
(43) Veröffentlichungstag der Anmeldung: 19.11.2003
(73) Patentinhaber: Wella Aktiengesellschaft, 64274 Darmstadt (DE)
(72) Erfinder: Lede, Michael, 63225 Langen (DE); Birkel, Susanne, 64285 Darmstadt (DE); Grasser, Beate, 65795 Hattersheim (DE); Keller, Walter, 64372 Ober-Ramstadt (DE)

(56) Entgegenhaltungen:
- EP-A- 0 357 186
- EP-A- 1 127 565
- EP-A- 1 199 064
- WO-A-01/43722
- WO-A-98/03148
- US-A- 4 393 076
- US-A- 5 585 379
- US-A- 5 925 295
- US-B1- 6 372 203
- PATENT ABSTRACTS OF JAPAN vol. 2000, no. 21, 3. August 2001 (2001-08-03) & JP 2001 097830 A (KOKYU ALCOHOL KOGYO CO LTD), 10. April 2001 (2001-04-10)

## Beschreibung

Gegenstand der Erfindung sind Haarstylinggele auf alkoholischer, im wesentlichen wasserfreier Basis mit einem Gehalt an einer Kombination aus alkohollöslichen, filmbildenden und haarfestigenden Polymeren, Gelbildnern, welche in der Lage sind, flüssige Alkohole zu verdicken, und mehr als 30 Gew.% an C1-4-Alkoholen.

Um dem menschlichen Haar Festigung und Halt zu geben, werden Haarbehandlungsmittel u.a. in Form von Haargelen eingesetzt. Haargele zeichnen sich in der Regel durch,eine gute Verteilbarkeit und Einarbeitbarkeit in das Haar aus. Das Haar läßt sich zu einer Frisur formen, welche nach Trocknung des Gels fixiert ist. Herkömmliche Haargele bestehen hauptsächlich aus Wasser, einem wasserlöslichen Verdickersystem und wasserlöslichen Stylingpolymeren (Harry's Cosmeticology, 8. Aufl., S. 647-650). In Ausnahmefällen können geringe Mengen an Alkohol zugesetzt sein, wobei der grundsätzliche mikro- und makroskopische Aufbau des Gels aber nicht verändert wird, da der Wassergehalt den Alkoholgehalt bei weitem überwiegt. Die herkömmlichen Gele auf Wasserbasis sind nicht immer vollkommen zufriedenstellend. Sie haben den Nachteil, dass sie eine sehr lange Trocknungszeit nach der Anwendung benötigen. Außerdem ist man bei der Formulierung auf wasserlösliche Stylingpolymere und deren inhärente Eigenschaften beschränkt. Es können nicht alle gewünschten anwendungstechnischen Eigenschaften erzielt werden, beispielsweise hinsichtlich Festigungsgrad, Feuchtigkeitsresistenz, Formbarkeit, Griff, Elastizität, Schutz oder Glanz des behandelten Haares. Der Optimierung des Wirkungsprofils sind Grenzen gesetzt.

Gele und verdickte Zusammensetzungen mit Gehalt an bestimmten alkohollöslichen Polymeren und mit Gehalt an alkoholische Systeme verdickenden Gelbildnern sowie mit mehr als 30% niederen Alkoholen werden beschrieben in der EP 1127565 A2 (Beispiele 3 und 5), US 4393076 (Beispiele), US 5585379 (Beispiele), US 5985295 (Beispiel 7), WO 01/43722 (Anspruch 7, Beispiele). In der JP 2001-097830 A werden alkoholhaltige Haarbehandlungsmittel beschrieben, welche als Lotion, Spray, Gel oder Schaum vorliegen können und 10 bis 60% Ethanol enthalten. Sofern es sich um Gele handelt, sind nur geringe Mengen Ethanol enthalten (Beispiele 10-11: 10% Ethanol).
In der EP 1199064 werden feste und formstabile Gele auf Basis einer Carrageenan enthaltenden wässrigen Grundlage beschrieben. Typische Wassergehalte sind 55-95%.

Es bestand daher die Aufgabe, ein Haarbehandlungsmittel zur Verfügung zu stellen, welches einerseits die anwendungstechnischen Vorteile eines herkömmlichen Haargels aufweist, gleichzeitig aber ein erweitertes oder verbessertes Wirkungsprofil aufweist.

Es wurde nun gefunden, dass die Aufgabe gelöst wird durch ein. Haarstylinggel mit Gehalt an einer Kombination aus
(A) mindestens einem alkohollöslichen, filmbildenden und haarfestigenden Polymer, welches bei 25°C zu mindestens 5 Gew.% in mindestens einem Alkohol mit 1 bis 4 C-Atomen löslich ist,
(B) mindestens einem flüssige Alkohole verdickenden Gelbildner und
(C) mehr als 30 Gew.% eines Alkohols mit 1 bis 4 C-Atomen wobei das Gel im wesentlichen wasserfrei ist und der Wassergehalt nicht größer als 10 Gew.% ist und wobei das Polymer (A) ausgewählt ist aus Copolymeren aus Acryl- oder Methacrylsäure und mindestens einem Monomer ausgewählt aus Acryl- oder Methacrylsäureestern und Acryl- oder Methacrylamiden, Copolymeren aus Crotonsäure und mindestens einem nichtionischen Comonomer, Copolymeren aus mindestens einem Acryl- oder Methacrylsäurester und mindestens einem davon verschiedenen nichtionischen Comonomeren, Copolymeren aus mindestens einem Maleinsäuremonoester und mindestens einem nichtionischen Comonomer, Vinylpyrrolidon/Vinylacetat Copolymeren und Polyvinylcaprolactam.

Das Polymer (A) wird vorzugsweise in einer Menge von 0,1 bis 20, besonders bevorzugt von 0,5 bis 15, ganz besonders bevorzugt von 1 bis 10 Gew.% eingesetzt. Der Gelbildner (B) wird vorzugsweise in einer Menge von 0,01 bis 20, besonders bevorzugt von 0,05 bis 10, ganz besonders bevorzugt von 0,1 bis 7 Gew.% eingesetzt. Der Alkohol (C) wird vorzugsweise in einer Menge von 50 bis 99, besonders bevorzugt von 70 bis 98 Gew.% eingesetzt.

Unter alkohollöslichen Polymeren werden solche Polymere verstanden, welche bei 25°C zu mindestens 5 Gew.% in mindestens einem Alkohol mit 1 bis 4 C-Atomen löslich sind. Unter filmbildenden und haarfestigenden Polymeren werden solche Polymere verstanden, welche bei Anwendung in 0,01 bis 5%iger Lösung eine haarfestigende Wirkung ausüben, insbesondere die Lockenstabilität (curl retention) gegenüber unbehandeltem Haar erhöhen.

Erfindungsgemäß geeignete haarfestigende Polymere können nichtionisch, anionisch, kationisch, zwitterionisch oder amphoter sein. Es kann ein synthetisches oder ein natürliches Polymer sein. Unter natürlichen Polymeren werden auch chemisch modifizierte Polymere natürlichen Ursprungs verstanden. Geeignet sind Copolymere aus mindestens einem ersten Monomer, ausgewählt aus Acrylsäure und Methacrylsäure und mindestens einem zweiten Monomer ausgewählt aus Acrylsäureestern, Methacrylsäureestern, Acrylamiden und Methacrylamiden. Bei den Estern kann es sich um Alkyl-, Aminoalkyl-, Monoalkylaminoalkyl oder Dialkylaminoalkylester handeln. Bei den Amiden kann es sich um unsubstiuierte Amide, um Monoalkylamide, um Dialkylamide, um Alkylaminoalkylamide oder um (Dialkylamino)alkylamide handeln. Die genannten Alkylgruppen weisen vorzugsweise 1 bis 30, besonders bevorzugt 1 bis 5 C-Atome auf. Weitere, geeignete haarfestigende Polymere sind Copolymere aus Crotonsäure und mindestens einem nichtionischen Comonomer. Bei dem nichtionischen Comonomer kann es sich um die oben genannten (Meth)acrylester oder (Meth)acrylamide oder um Vinylester handeln. Geeignete Vinylester sind Ester von Vinylalkohol und C1- bis C12-Carbonsäuren. Weitere, geeignete haarfestigende Polymere sind Copolymere aus mindestens einem Acryl- oder Methacrylsäurester und mindestens einem nichtionischen Comonomeren. Bei dem nichtionischen Comonomer kann es sich um die oben genannten (Meth)acrylester oder (Meth)acrylamide, um die oben genannten Vinylester oder um Vinyllactame, z.B. Vinylpyrrolidon oder Vinylcaprolactam handeln. Weitere, geeignete haarfestigende Polymere sind Copolymere aus mindestens einem Maleinsäuremonoester und mindestens einem nichtionischen Comonomer. Geeignete Maleinsäuremonoester sind C1- bis C7-Alkylester. Geeignete nichtioniche Comonomere sind die oben genannten oder Alkylvinylether, insbesondere Methylvinylether. Weitere, geeignete haarfestigende Polymere sind Polyvinylcaprolactame. Weitere, geeignete haarfestigende Polymere sind Copolymere aus mindestens einem ersten Monomer, ausgewählt aus Vinyllactamen, z.B. Vinylpyrrolidon oder Vinylcaprolactam und mindestens einem zweiten Monomer, ausgewählt aus den oben genannten Vinylestern, insbesondere Vinylacetat.

Bevorzugte haarfestigende Polymere sind ausgewählt aus Polyvinylcaprolactam (z.B. Luviskol® Plus), Vinylpyrrolidon/Vinylacetat Copolymer (z.B. Luviskol® VA 37), Terpolymere aus Vinylpyrrolidon, Vinylacetat und Vinylpropionat (z.B. Luviskol® VAP 343), Copolymere der Acrylsäure oder Methacrylsäure mit Monomeren ausgewählt aus Acrylsäure- oder Methacrylsäureestern oder -amiden, insbesondere Acrylsäure/Ethylacrylat/N-tert-butylacrylat Copolymer (z.B. Ultrahold® 8), Octylacrylamid/Acrylsäure/t-Butylaminoethylmethacrylat/Hydroxypropylmethacrylat Copolymer (z.B. Amphomer® ), t-Butylacrylat/Ethylacrylat/Methacrylsäure Copolymer (z.B. Luvimer® 100 P), Vinylacetat/Crotonsäure Copolymer (z.B. Luviset® CA66 oder Resyn 28 1310), Vinylacetat/Crotonsäure/Vinylneodecanoat Copolymer (z.B. Resyn 28 2930), Methylvinylether/Maleinsäuremonoalkylester Copolymere (z.B. Gantrez® ES 425) und Vinylpyrrolidon/Vinylcaprolactam/Dimethylaminopropylmethacrylamid Copolymer (z.B. Aquaflex® SF40).

Enthalten die haarfestigenden Polymere Säuregruppen, so sind die Säuregruppen vorzugsweise zumindest teilweise mit kosmetisch verträglichen Basen neutralisiert. Der Neutralisationsgrad liegt bevorzugt bei 50 bis 100%, besonders bevorzugt bei 80-100%. Als Neutralisationsmittel können für kosmetische Zwecke geeignete organische oder anorganische Basen verwendet werden. Beispiele für Basen sind Aminoalkohole wie z.B. Aminomethylpropanol (AMP), Triethanolamin oder Monoethanolamin und Ammoniak, NaOH und andere.

Geeignete Gelbildner (B) sind solche, welche in C1-4-Alkoholen, insbesondere in Ethanol oder Isopropanol dispergierbar oder löslich sind und deren Zusatz zu diesen Alkoholen eine Viskositätserhöhung bewirkt. Geeignet sind z.B. Salze von Fettsäuren, Kieselgur, Polysaccharidether, Ester von Polysacchariden oder organolösliche, insbesondere ethanollösliche Chitin- oder Chitosanderivate. Geeignete Fettsäuresalze sind insbesondere Salze der Stearinsäure, z.B. Natrium-, Kalium- oder Magnesiumstearat. Geeignete Polysaccharidether sind insbesondere Hydroxyalkylpolysaccharide, z.B. Hydroxyalkylcellulosen, Hydroxyalkylmethylcellulosen oder Hydroxyalkylguar, wobei die Alkylgruppen 1 bis 4 C-Atome aufweisen. Ein besonders bevorzugter Gelbildner ist Hydroxypropylcellulose.

Geeignete flüssige Alkohole (C) sind ein- oder mehrwertige Alkohole, welche bei Raumtemperatur (20°C) flüssig sind und 1 bis 4 C-Atome aufweisen. Als Alkohole können insbesondere die für kosmetische Zwecke üblicherweise verwendeten niederen Alkohole wie z.B. Ethanol, Isopropanol, Glycerin, Ethylenglykol oder Propylenglykol enthalten sein. Besonders bevorzugt sind einwertige Alkohole mit 2 bis 4 C-Atomen, insbesondere Ethanol und Isopropanol. Das Haargel ist vorzugsweise im wesentlichen wasserfrei, es kann aber zur Verbesserung der Löslichkeit weiterer Inhaltsstoffe geringe Wassermengen enthalten, wobei der Alkoholgehalt aber den Wassergehalt deutlich übersteigt. Im wesentlichen wasserfrei bedeutet, dass der Wassergehalt nicht größer als 10 Gew.%, vorzugsweise nicht größer als 5 Gew.% ist.

Bei dem erfindungsgemäßen Haargel beträgt die Viskosität vorzugsweise 500 bis 50.000 mPa s, besonders bevorzugt von 1.000 bis 15.000 mPa s, gemessen als dynamische Viskositätsmessung mit einem HAAKE Rotationsviskosimeter VT501 bei 25°C mit einem Prüfkörper nach DIN 53019 (SV-DIN) und einer Schergeschwindigkeit von 12,9 s⁻¹ (Drehzahlstufe 5, Reihe B).

Das erfindungsgemäße Mittel kann darüber hinaus die für Haarbehandlungsmittel üblichen, vorzugsweise alkohollöslichen Zusatzbestandteile enthalten, z.B. Netzmittel oder Emulgatoren aus den Klassen der nichtionischen, anionischen, kationischen oder amphoteren oberflächenaktiven Tenside in einer Menge von 0,1 bis 15 Gew.%; Feuchthaltemittel; Parfümöle in einer Menge von 0,01 bis 1 Gew.%; Trübungsmittel, wie z.B. Ethylenglykoldistearat, in einer Menge von etwa 0,2 bis 5,0 Gew.%; Perlglanzmittel, wie z.B. ein Gemisch aus Fettsäuremonoalkylolamid und Ethylenglykoldistearat, in einer Menge von etwa 1,0 bis 10 Gew.%; bakterizide und fungizide Wirkstoffe wie z.B. 2,4,4-Trichlor-2-hydroxydiphenylether oder Methylchlorisothiazolin, in einer Menge von 0,01 bis 1,0 Gew.%, Puffersubstanzen, wie z.B. Natriumcitrat oder Natriumphosphat, in einer Menge von 0,1 bis 1,0 Gew.%; Anfärbestoffe, wie z.B. Fluorescein Natriumsalz, in einer Menge von etwa 0,1 bis 1,0 Gew.%; Pflegestoffe, wie z.B. Pflanzen- und Kräuterextrakte, Protein- und Seidenhydrolysate, Lanolinderivate, in einer Menge von 0,1 bis 5 Gew.%; physiologisch verträgliche Silikonderivate, wie z.B. flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Glanzgeber, Vitamine, Weichmacher, Kämmbarkeitsverbesserer, rückfettende Agenzien, in einer Menge von 0,01 bis 4 Gew.%.

Die erfindungsgemäßen alkoholischen Gele zeichen sich durch einen hohen Festigungsgrad, eine schnelle Trocknung und eine angenehme Kühlwirkung aus. Sie können in klarer Form, z.B. unter Verwendung von Hydroxyalkylsacchariden als Verdicker, oder in opaker, weisser Form, z.B. unter Verwendung von Fettsäuresalzen als Verdicker hergestellt werden.

Die nachfolgenden Beispiele sollen den Gegenstand der Erfindung näher erläutern.

### Beispiele

### Beispiel 1: Klares Haargel

| | |
|---|---|
| 3 g | Acrylsäure/Ethylacrylat/N-tert.-Butylacrylat Copolymer (Ultrahold® 8) |
| 7 g | Hydroxypropylcellulose (Klucel® HF) |
| 0,22 g | Aminomethylpropanol |
| 0, 2 g | Parfüm |
| ad 100 g | Ethanol |

Viskosität: 4600 mPa s, gemessen als dynamische Viskositätsmessung mit einem HAAKE Rotationsviskosimeter VT501 bei 25°C mit einem Prüfkörper nach DIN 53019 (SV-DIN) und einer Schergeschwindigkeit von 12,9 s⁻¹ (Drehzahlstufe 5, Reihe B).

### Beispiel 2

| | |
|---|---|
| 3 g | Acrylsäure/Ethylacrylat/N-tert.-Butylacrylat Copolymer (Ultrahold® 8) |
| 7 g | Hydroxypropylcellulose (Klucel® HF) |
| 0,22 g | Aminomethylpropanol |
| 0, 2 g | Parfüm |
| ad 100 g | Ethanol |

### Beispiel 3

| | |
|---|---|
| 5 g | Octylacrylamid/Acrylsäure/Butylaminoethylmethacry-lat/Hydroxypropylmethacrylat Copolymer (Amphomer® ) |
| 2 g | Hydroxypropylcellulose (Klucel® HF) |
| 0,52 g | Aminomethylpropanol |
| 0,2 g | Parfüm |
| ad 100 g | Ethanol |

### Beispiel 4

| | |
|---|---|
| 10 g | Octylacrylamid/Acrylsäure/Butylaminoethylmethacrylat/Hydroxypropylmethacrylat Copolymer (Amphomer® ) |
| 3 g | Hydroxypropylcellulose (Klucel® HF) |
| 0,92 g | Aminomethylpropanol |
| 0,2 g | Parfüm |
| ad 100 g | Ethanol |

### Beispiel 5

| | |
|---|---|
| 8 g | Vinylacetat/Crotonsäure Copolymer (Luviset® CA 66) |
| 5 g | Hydroxypropylcellulose (Klucel® HF) |
| 0,66 g | Aminomethylpropanol |
| 0,2 g | Parfüm |
| ad 100 | g Ethanol |

### Beispiel 6

| | |
|---|---|
| 5 g | Vinylacetat/Crotonsäure Copolymer (Luviset® CA 66) |
| 5 g | t-Butylacrylat/Ethylacrylat/Methacrylsäure Copolymer (Luvimer® 100 P) |
| 3 g | Hydroxypropylcellulose (Klucel® HF) |
| 0,41 g | Aminomethylpropanol |
| 0,2 g | Parfüm |
| ad 100 g | Ethanol |

### Beispiel 7

| | |
|---|---|
| 3 g | Vinylacetat/Crotonsäure Copolymer (Luviset® CA 66) |
| 5 g | Vinylacetat/Crotonsäure/Vinylneodecanoat Copolymer (Resyn 28 2930) |
| 2 g | Hydroxypropylcellulose (Klucel® HF) |
| 0,41 g | Aminomethylpropanol |
| 0,2 g | Parfüm |
| ad 100 g | Ethanol |

### Beispiel 8: Klares Haargel

| | |
|---|---|
| 3 g | Acrylsäure/Ethylacrylat/N-tert.-Butylacrylat Copolymer (Ultrahold® 8) |
| 2 g | Hydroxypropylmethylcellulose (Methocel® 311) |
| 0,22 g | Aminomethylpropanol |
| 0,2 g | Parfüm |
| 5 g | Wasser |
| ad 100 g | Ethanol |

### Beispiel 9: Weißes Haargel (nicht Teil der Erfindung)

| | |
|---|---|
| 3 g | Acrylsäure/Ethylacrylat/N-tert.-Butylacrylat Copolymer (Ultrahold® 8) |
| 2 g | Natriumstearat |
| 0,22 g | Aminomethylpropanol |
| 0,2 g | Parfüm |
| ad 100 g | Ethanol |

## Patentansprüche

1. Haarstylinggel mit Gehalt an einer Kombination aus
(A) mindestens einem alkohollöslichen, filmbildenden und haarfestigenden Polymer, welches bei 25°C zu mindestens 5 Gew.% in mindestens einem Alkohol mit 1 bis 4 C-Atomen löslich ist,
(B) mindestens einem flüssige Alkohole verdickenden Gelbildner und
(C) mehr als 30 Gew.% eines Alkohols mit 1 bis 4 C-Atomen, wobei das Gel im wesentlichen wasserfrei ist und der Wassergehalt nicht größer als 10 Gew.% ist und wobei das Polymer (A) ausgewählt ist aus Copolymeren aus Acryl- oder Methacrylsäure und mindestens einem Monomer ausgewählt aus Acryl- oder Methacrylsäureestern und Acryl- oder Methacrylamiden, Copolymeren aus Crotonsäure und mindestens einem nichtionischen Comonomer, Copolymeren aus mindestens einem Acryl- oder Methacrylsäurester und mindestens einem davon verschiedenen nichtionischen Comonomeren, Copolymeren aus mindestens einem Maleinsäuremonoester und mindestens einem nichtionischen Comonomer, Vinylpyrrolidon/Vinylacetat Copolymeren und Polyvinylcaprolactam.

2. Gel nach Anspruch 1, **dadurch gekennzeichnet, dass** das Polymer (A) in einer Menge von 0,1 bis 20 Gew.% und der Gelbildner (B) in einer Menge von 0,01 bis 20 Gew.% enthalten sind.

3. Gel nach einem der Ansprüche 1 bis 2, **dadurch gekennzeichnet, dass** der Gelbildner ausgewählt ist aus Stearinsäuresalzen, Polysaccharidethern und Polysaccharidestern.

4. Gel nach einem der Ansprüche 1 bis 3, **gekennzeichnet durch** einen Gehalt an
(A) 1 bis 15 Gew.% mindestens eines alkohollöslichen, filmbildenden und haarfestigenden Polymers, ausgewählt aus Copolymeren aus Acryl- oder Methacrylsäure und mindestens einem Monomer ausgewählt aus Acryl- oder Methacrylsäureestern und Acryl- oder Methacrylamiden, Copolymeren aus Crotonsäure und mindestens einem nichtionischen Comonomer, Copolymeren aus mindestens einem Acryl- oder Methacrylsäurester und mindestens einem davon verschiedenen nichtionischen Comonomeren, Copolymeren aus mindestens einem Maleinsäuremonoester und mindestens einem nichtionischen Comonomer, Vinylpyrrolidon/Vinylacetat Copolymeren und Polyvinylcaprolactam,
(B) 0,1 bis 7 Gew.% mindestens eines Ethanol verdickenden Gelbildners, ausgewählt aus Stearinsäuresalzen, Hydroxyalkylcellulosen und Celluloseestern und
(C) 50 bis 98 Gew.% Ethanol und
(D) 0 bis 10 Gew.% Wasser.

## Claims

1. Hair-styling gel with a content of a combination of
(A) at least one alcohol-soluble, film-forming and hair-setting polymer which is soluble at 25°C to at least 5% by weight in at least one alcohol having 1 to 4 carbon atoms,
(B) at least one gel former which thickens liquid alcohols and
(C) more than 30% by weight of an alcohol having 1 to 4 carbon atoms, where the gel is essentially water free and the water content is not greater than 10% by weight and where the polymer (A) is chosen from copolymers of acrylic acid or methacrylic acid and at least one monomer chosen from acrylic or methacrylic acid esters and acrylamides or methacrylamides, copolymers of crotonic acid and at least one nonionic comonomer, copolymers of at least one acrylic or methacrylic acid ester and at least one nonionic comonomer different therefrom, copolymers of at least one maleic acid monoester and at least one nonionic comonomer, vinylpyrrolidone/vinyl acetate copolymers and polyvinylcaprolactam.

2. Gel according to Claim 1, **characterized in that** the polymer (A) is present in an amount of from 0.1 to 20% by weight and the gel former (B) is present in an amount of from 0.01 to 20% by weight.

3. Gel according to one of Claims 1 to 2, **characterized in that** the gel former is chosen from stearic acid salts, polysaccharide ethers and polysaccharide esters.

4. Gel according to one of Claims 1 to 3, **characterized by** a content of
(A) 1 to 15% by weight of at least one alcohol-soluble, film-forming and hair-setting polymer chosen from copolymers of acrylic acid or methacrylic acid and at least one monomer chosen from acrylic or methacrylic acid esters and acrylamides or methacrylamides, copolymers of crotonic acid and at least one nonionic comonomer, copolymers of at least one acrylic or methacrylic acid ester and at least one nonionic comonomer different therefrom, copolymers of at least one maleic acid monoester and at least one nonionic comonomer, vinylpyrrolidone/vinyl acetate copolymers and polyvinylcaprolactam,
(B) 0.1 to 7% by weight of at least one gel former which thickens ethanol, chosen from stearic acid salts, hydroxyalkylcelluloses and cellulose esters and
(C) 50 to 98% by weight of ethanol and
(D) 0 to 10% by weight of water.

## Revendications

1. Gel de coiffage à teneur en une association de
(A) au moins un polymère soluble dans un alcool, filmogène et fixant les cheveux, qui, à 25 °C, est soluble à raison d'au moins 5 % en poids dans au moins un alcool ayant de 1 à 4 atomes de carbone,
(B) au moins un agent gélifiant épaississant des alcools liquides et
(C) plus de 30 % en poids d'un alcool ayant de 1 à 4 atomes de carbone,
le gel étant pratiquement anhydre et la teneur en eau n'excédant pas 10 % en poids et le polymère (A) étant choisi parmi les copolymères d'acide acrylique ou méthacrylique et d'au moins un monomère choisi parmi les esters d'acide acrylique ou méthacrylique et les acryl- ou méthacrylamides, les copolymères d'acide crotonique et d'au moins un comonomère non ionique, les copolymères d'au moins un ester d'acide acrylique ou méthacrylique et d'au moins un comonomère non ionique différent de celui-ci, les copolymères d'au moins un monoester d'acide maléique et d'au moins un comonomère non ionique, les copolymères vinylpyrrolidone/ acétate de vinyle et le polyvinylcaprolactame.

2. Gel selon la revendication 1, **caractérisé en ce que** le polymère (A) est contenu en une quantité de 0,1 à 20 % en poids et l'agent gélifiant (B) est contenu en une quantité de 0,01 à 20 % en poids.

3. Gel selon la revendication 1 ou 2, **caractérisé en ce que** le l'agent gélifiant est choisi parmi les sels d'acide stéarique, les éthers de polysaccharides et les esters de polysaccharides.

4. Gel selon l'une quelconque des revendications 1 à 3, **caractérisé par** une teneur de
(A) 1 à 15 % en poids en au moins un polymère soluble dans un alcool, filmogène et fixant les cheveux, choisi parmi les copolymères d'acide acrylique ou méthacrylique et d'au moins un monomère choisi parmi les esters d'acide acrylique ou méthacrylique et les acryl- ou méthacrylamides, les copolymères d'acide crotonique et d'au moins un comonomère non ionique, les copolymères d'au moins un ester d'acide acrylique ou méthacrylique et d'au moins un comonomère non ionique différent de celui-ci, les copolymères d'au moins un monoester d'acide maléique et d'au moins un comonomère non ionique, les copolymères vinylpyrrolidone/acétate de vinyle et le polyvinylcaprolactame
(B) 0,1 à 7 % en poids en au moins un agent gélifiant épaississant l'éthanol, choisi parmi les sels d'acide stéarique, les hydroxyalkylcelluloses et les esters de cellulose, et
(C) 50 à 98 % en poids en éthanol et
(D) 0 à 10 % en poids en eau,
